(19) Europäisches Patentamt
European Patent Office
Office européen des brevets

(11) **EP 3 455 631 B1**

(12) **EUROPEAN PATENT SPECIFICATION**

(45) Date of publication and mention
of the grant of the patent:
**24.06.2020 Bulletin 2020/26**

(21) Application number: **17725195.6**

(22) Date of filing: **09.05.2017**

(51) Int Cl.:
**G01N 33/574** (2006.01)    **G16H 50/30** (2018.01)

(86) International application number:
**PCT/EP2017/061089**

(87) International publication number:
**WO 2017/194556 (16.11.2017 Gazette 2017/46)**

(54) **METHODS FOR CLASSIFYING PATIENTS WITH A SOLID CANCER**

VERFAHREN ZUR KLASSIFIZIERUNG VON PATIENTEN MIT EINEM SOLIDEM KREBS

PROCÉDÉS POUR LA CLASSIFICATION DE PATIENTS ATTEINTS D'UN CANCER SOLIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.05.2016 EP 16305536**

(43) Date of publication of application:
**20.03.2019 Bulletin 2019/12**

(73) Proprietors:
• **INSERM (Institut National de la Santé et de la Recherche Médicale)**
**75013 Paris (FR)**
• **SORBONNE UNIVERSITE**
**75006 Paris (FR)**
• **Université Paris Descartes**
**75006 Paris (FR)**
• **Université Paris Diderot - Paris 7**
**75013 Paris (FR)**
• **Assistance Publique - Hôpitaux de Paris**
**75004 Paris (FR)**

(72) Inventors:
• **GALON, Jérôme**
**75006 Paris (FR)**
• **MLECNIK, Bernhard**
**75006 Paris (FR)**
• **PAGES, Franck**
**75006 Paris (FR)**

(74) Representative: **Cabinet Becker et Associés**
**25, rue Louis le Grand**
**75002 Paris (FR)**

(56) References cited:
**WO-A1-2010/120814      WO-A1-2013/107900**
**WO-A1-2013/186374      WO-A1-2014/023706**
**WO-A1-2015/007625      WO-A2-2014/009535**

• **JÉRÔME GALON ET AL: "Cancer classification using the Immunoscore: a worldwide task force", JOURNAL OF TRANSLATIONAL MEDICINE, BIOMED CENTRAL, LONDON, GB, vol. 10, no. 1, 3 October 2012 (2012-10-03), page 205, XP021134189, ISSN: 1479-5876, DOI: 10.1186/1479-5876-10-205**
• **FABIENNE HERMITTE: "Biomarkers immune monitoring technology primer: Immunoscore Colon", JOURNAL FOR IMMUNOTHERAPY OF CANCER, vol. 232, no. Suppl abstr, 20 September 2016 (2016-09-20), page 2, XP055314364, DOI: 10.1186/s40425-016-0161-x**

**Description**

**FIELD OF THE INVENTION:**

The present invention relates to methods for classifying patients suffering from a solid cancer, particularly to methods for the prognosis of the survival time of a patient suffering from a solid cancer and/or to methods for assessing the responsiveness of a patient suffering from a solid cancer to antitumoral treatment.

**BACKGROUND OF THE INVENTION:**

As explained in detail by Galon et al (Cancer classification using the Immunoscore: a worldwide task force J Transl Med. 2012; 10: 205), prediction of clinical outcome in cancer is usually achieved by histopathological evaluation of tissue samples obtained during surgical resection of the primary tumor. Traditional tumor staging (AJCC/UICC-TNM classification) summarizes data on tumor burden (T), presence of cancer cells in draining and regional lymph nodes (N) and evidence for metastases (M). However, it is now recognized that clinical outcome can significantly vary among patients within the same stage. The current classification provides limited prognostic information, and does not predict response to therapy. Recent literature has alluded to the importance of the host immune system in controlling tumor progression. Thus, evidence supports the notion to include immunological biomarkers, implemented as a tool for the prediction of prognosis and response to therapy. Accumulating data, collected from large cohorts of human cancers, has demonstrated the impact of immune-classification, which has a prognostic value that may add to the significance of the AJCC/UICC TNM-classification. It is therefore imperative to begin to incorporate the 'Immunoscore' into traditional classification, thus providing an essential prognostic and potentially predictive tool. Introduction of this parameter as a biomarker to classify cancers, as part of routine diagnostic and prognostic assessment of tumors, will facilitate clinical decision-making including rational stratification of patient treatment.

Numerous patent applications have described methods the prognosis of the survival time of a patient suffering from a solid cancer and/or methods for assessing the responsiveness of a patient suffering from a solid cancer to antitumoral treatment, by measuring immunological biomarkers. One can cite for example: WO2015007625, WO2014023706, WO2014009535, WO2013186374, WO2013107907, WO2013107900, WO2012095448, WO2012072750 and WO2007045996. All these methods provide good results. As explained in the review Galon et al. Immunity 39, 2013, 11-26, prognostic and predictive immune signatures are largely overlapping.

WO2010/120814 discloses, *inter alia*, methods relying on a quartile statistical analysis for prognosing patients suffering from inflammatory bowel disease (IBD).

The present inventors managed to further increase the accuracy of the methods for the prognosis of the survival time of a patient suffering from a solid cancer and of the methods for assessing the responsiveness of a patient suffering from a solid cancer to antitumoral treatment.

**SUMMARY AND DETAILED DESCRIPTION OF THE INVENTION:**

The scope of the present invention is defined by the claims and any information that does not fall within the claims is provided for information only.

The present invention relates to an in vitro method for the prognosis of the survival time of a patient suffering from a solid cancer, which method comprises the following steps:

a) detecting and quantifying two or more biological markers indicative of the status of the immune response of said patient against said cancer, wherein each biological marker indicative of the status of the immune response is detected *in vitro* quantified in a tumor sample obtained from said patient;
b) comparing each values obtained at step a) for said two or more biological markers with a distribution of values obtained for each of said two or more biological markers from a reference group of patients suffering from said cancer;
c) determining for each values obtained at step a) for said two or more biological markers the percentile of the distribution to which the values obtained at step a) correspond;
d) calculating the arithmetic mean value or the median value of percentile; and
e) comparing the arithmetic mean value or the median value of percentile obtained at step d) with a predetermined reference arithmetic mean value or a predetermined median value of percentile, which predetermined reference value is correlated with survival time. Typically, the survival time is Disease-free survival (DFS), Progression Free Survival (PFS), Disease Specific Survival (DSS), or Overall survival (OS).

The present invention also relates to an in vitro method for assessing the responsiveness of a patient suffering from a solid cancer to an antitumoral treatment, which method comprises the following steps:

a) detecting and quantifying two or more biological markers indicative of the status of the immune response of said patient against said cancer, wherein each biological marker indicative of the status of the immune response is detected *in vitro* quantified in a tumor sample obtained from said patient;

b) comparing each values obtained at step a) for said two or more biological markers with a distribution of values obtained for each of said two or more biological markers from a reference group of patients suffering from said cancer;

c) determining for each values obtained at step a) for said two or more biological markers the percentile of the distribution to which the values obtained at step a) correspond;

d) calculating the arithmetic mean value or the median value of percentile; and

e) comparing the arithmetic mean value or the median value of percentile obtained at step d) with a predetermined reference arithmetic mean value or a predetermined median value of percentile, which predetermined reference value is correlated with response to said antitumoral treatment.

The term "arithmetic mean value" should be understood broadly and encompasses the "classical *"arithmetic mean* of a sample which is the sum of the sampled values divided by the number of items in the sample and also the weighted arithmetic mean:

$$\bar{x} = \frac{\sum_{i=1}^{n} w_i \cdot x_i}{\sum_{i=1}^{n} w_i}.$$

The weights wi may represent a measure for the reliability of the influence upon the mean by the respective values.

The median value is particularly adapted when many (typically more than 5, 10, 15, 200) biological markers are quantified.

The methods of the invention have unique and multiple advantages:

Said methods perform very significantly and have a strong discriminatory power because the biomarkers considered in these methods have important specific characteristics.

The biomarkers considered are immune biomarkers. In particular, the biomarkers are adaptive immune biomarkers. One of their essential characteristics is that these immune biomarkers are associated with the time of recurrence, with the time before death, with the time before the response to treatment, with the amplitude of response to treatment, and with the prolonged time during which the patient is responding to treatment.

One of the advantages of the methods of the invention is that the level of intensity, expression, density, quantity of each biomarker is associated with the length, with the time of survival, with the prolonged survival, and the prolonged response to the treatment.

The methods of the invention apply because the higher or the lower the levels of the biomarkers are, the longer or the shorter the survival is and the better or the worse the response to the treatment is.

One of the essential novel elements associated with the great performance of the methods is that the methods are independent of the clinical data and follow-up of the patients. The mean Percentile can be calculated based on the raw and normalized data of the biomarker, independently of the outcome of the patients.

A comprehensive adaptive immune reaction is characterized by a set of biomarkers, that when co-enriched, the biomarkers are improving the stratification of the patients into categories of prolonged survival and better response to treatment. The mean of the percentile values of all biomarkers, weighted for each biomarker, allow a very accurate stratification of the patients into risk categories.

Typically, the reference group of patients suffering from said cancer comprises at least: 100, 200, 300, 400, 500, 1000 or 2000 patients.

Typically, the methods of the invention apply to various organs of cancer origin (such as breast, colon, rectum, lung, head and neck, bladder, ovary, prostate), and also to various cancer cell types (adenocarcinoma, squamous cell carcinoma, large cell cancer, melanoma, etc).

Typically the patient subjected to the above method may suffer from a solid cancer selected from the group consisting adrenal cortical cancer, anal cancer, bile duct cancer (e.g. periphilar cancer, distal bile duct cancer, intrahepatic bile duct cancer), bladder cancer, bone cancer (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, multiple myeloma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating lobular carcinoma, lobular carcinoma in situ, gynecomastia), cervical cancer, colorectal cancer, endometrial cancer (e.g. endometrial adenocarcinoma, adenocanthoma, papillary serous adnocarcinoma, clear cell), esophagus cancer, gallbladder cancer (mucinous adenocarcinoma, small cell carcinoma), gastrointestinal carcinoid tumors (e.g. choriocarcinoma, chorioadenoma destruens), Kaposi's sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma), vaginal cancer, vulvar cancer, and uterine cancer (e.g. uterine leiomyosarcoma).

In a preferred embodiment, the cancer is colorectal cancer.

As used herein, the term "tumor tissue sample" means any tissue tumor sample derived from the patient. Said tissue sample is obtained for the purpose of the in vitro evaluation. In some embodiments, the tumor sample may result from the tumor resected from the patient. In some embodiments, the tumor sample may result from a biopsy performed in the primary tumour of the patient or performed in metastatic sample distant from the primary tumor of the patient. For example an endoscopical biopsy performed in the bowel of the patient affected by a colorectal cancer. Typically the tumor tissue sample is fixed in formalin and embedded in a rigid fixative, such as paraffin (wax) or epoxy, which is placed in a mould and later hardened to produce a block which is readily cut. Thin slices of material can be then prepared using a microtome, placed on a glass slide and submitted e.g. to immunohistochemistry (using an IHC automate such as BenchMark® XT, for obtaining stained slides). The tumour tissue sample can be used in microarrays, called as tissue microarrays (TMAs). TMA consists of paraffin blocks in which up to 1000 separate tissue cores are assembled in array fashion to allow multiplex histological analysis. This technology allows rapid visualization of molecular targets in tissue specimens at a time, either at the DNA, RNA or protein level. TMA technology is described in WO2004000992, US8068988, Olli et al 2001 Human Molecular Genetics, Tzankov et al 2005, Elsevier; Kononen et al 1198; Nature Medicine.

In some embodiments, the tumor tissue sample encompasses (i) a global primary tumor (as a whole), (ii) a tissue sample from the center of the tumor, (iii) a tissue sample from the tissue directly surrounding the tumor which tissue may be more specifically named the "invasive margin" of the tumor, (iv) lymphoid islets in close proximity with the tumor, (v) the lymph nodes located at the closest proximity of the tumor, (vi) a tumor tissue sample collected prior surgery (for follow-up of patients after treatment for example), and (vii) a distant metastasis.

As used herein the "invasive margin" has its general meaning in the art and refers to the cellular environment surrounding the tumor. In some embodiments, the tumor tissue sample, irrespective of whether it is derived from the center of the tumor, from the invasive margin of the tumor, or from the closest lymph nodes, encompasses pieces or slices of tissue that have been removed from the tumor center of from the invasive margin surrounding the tumor, including following a surgical tumor resection or following the collection of a tissue sample for biopsy, for further quantification of one or several biological markers, notably through histology or immunohistochemistry methods, through flow cytometry methods and through methods of gene or protein expression analysis, including genomic and proteomic analysis. The tumor tissue sample can, of course, be subjected to a variety of well-known post-collection preparative and storage techniques (e.g., fixation, storage, freezing, etc.). The sample can be fresh, frozen, fixed (e.g., formalin fixed), or embedded (e.g., paraffin embedded). In some embodiments, when the quantification of the number of tumor-draining lymph nodes is performed in the ressected tumor, the tumor tissue sample results from said ressected tumor and encompasses the center of the tumor, and optionally the invasive margin of the tumor. In said embodiments, the quantification of the marker of the immune adaptive response is typically performed by immunohistochemistry (IHC) a described after. In some embodiments, when the quantification of the number of tumor-draining lymph nodes is performed is determined by imagery, the tumor tissue sample results from a biopsy. In said embodiments, the quantification of the marker of the immune adaptive response is typically performed by determining the expression level of at least one gene.

Typically, the tumor sample may be selected from the group consisting of (i) a global primary tumor (as a whole), (ii) a tissue sample from the center of the tumor, (iii) a tissue sample from the tissue directly surrounding the tumor which tissue may be more specifically named the "invasive margin" of the tumor, (iv) the lymph nodes located at the closest proximity of the tumor or a tertiary lymphoid structure induced by the tumor, (v) a tumor biopsie performed at any time and typically prior surgery, and (vi) a distant metastasis.

In a preferred embodiment the two or more biological markers are quantified in the center of the tumor and/or in the invasive margin of the tumor.

In a preferred embodiment the two or more biological markers are quantified in the center of the tumor and/or in the invasive margin of the tumor.

The sample can be fresh, frozen, fixed (e.g., formalin fixed), or embedded (e.g., paraffin embedded). In a particular embodiment the tumour sample results from biopsy performed in a tumour of the patient.

An example is an endoscopical biopsy performed in the bowel of the patient suffering from colorectal cancer or suspected to suffer from colorectal cancer.

As intended herein, a "biological marker" consists of any detectable, measurable and quantifiable parameter that is indicative of the status of the immune response of the cancer patient against the tumor. Biological markers include the presence of, or the number or density of, cells from the immune system at the tumor site.

Biological markers also include the presence of, or the amount of proteins specifically produced by cells from the immune system at the tumor site.

Biological markers also include the presence of, or the amount of, any biological material that is indicative of the expression level of genes related to the raising of a specific immune response of the host, at the tumor site. Thus, biological markers include the presence of, or the amount of, messenger RNA (mRNA) transcribed from genomic DNA encoding proteins which are specifically produced by cells from the immune system, at the tumor site.

Biological markers thus include surface antigens that are specifically expressed by cells from the immune system, including by B lymphocytes, T lymphocytes, monocytes/macrophages dendritic cells, NK cells, NKT cells, and NK-DC cells., that are recruited within the tumor tissue or at its close proximity, including within the invasive margin of the tumor and in the closest lymph nodes, or alternatively mRNA encoding for said surface antigens.

Illustratively, surface antigens of interest used as biological markers include CD3, CD4, CD8 and CD45RO that are expressed by T cells or T cell subsets.

For example, if the expression of the CD3 antigen, or the expression of the mRNA thereof, is used as a biological marker, the quantification of this biological marker, at step a) of the method according to the invention, is indicative of the level of the adaptive immune response of the patient involving all T lymphocytes and NKT cells.

For instance, if the expression of the CD8 antigen, or the expression of the mRNA thereof, is used as a biological marker, the quantification of this biological marker, at step a) of the method according to the invention, is indicative of the level of the adaptive immune response of the patient involving cytotoxic T lymphocytes.

For example, if the expression of the CD45RO antigen, or the expression of the mRNA thereof, is used as a biological marker, the quantification of this biological marker, at step a) of the method according to the invention, is indicative of the level of the adaptive immune response of the patient involving memory T lymphocytes or memory effector T lymphocytes.

Yet illustratively, proteins used as biological markers also include cytolytic proteins specifically produced by cells from the immune system, like perforin, granulysin and also granzyme-B.

Numerous patent applications have described a large number of biological markers indicative of the status of the immune response which could be used in the methods of the invention.

Typically, one can used the biological markers indicative of the status of the immune response described in WO2015007625, WO2014023706, WO2014009535, WO2013186374, WO2013107907, WO2013107900, WO2012095448, WO2012072750 and WO2007045996.

Typically a combination of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50 distinct biological markers may be quantified, preferably a combination of 2, 3, 4, 5, 6, 7, 8, 9, or 10 biological markers and more preferably a combination of 2, 3, 4, 5, or 6, biological markers.

In a preferred embodiment, the biological markers indicative of the status of the immune response are those described in WO2007045996.

Typically, the biological markers which may be used are the cell density of cells from the immune system. In a preferred embodiment the two or more biological markers comprise the density of CD3+ cells, the density of CD8+ cells, the density of CD45RO+ cells, the density of GZM-B+ cells and/or the density of B cells.

In a most preferred embodiment the two or more biological markers comprise the density of CD3+ cells and the density of CD8+ cells, the density of CD3+ cells and the density of CD45RO+ cells, the density of CD3+ cells the density of GZM-B+ cells, the density of CD8+ cells and the density of CD45RO+ cells, the density of CD8+ cells and the density of GZM-B+ cells; or the density of CD45RO+ cells and the density of GZM-B+ cells.

The density of B-cells may also be measured (see WO2013107900 and WO2013107907). The density of DC cells may also be measured (see WO2013107907).

In a preferred embodiment the density of cells from the immune system are quantified in the center of the tumor and/or in the invasive margin of the tumor.

In a most preferred embodiment, the two or more biological markers comprise the density of CD3+ cells in center of the tumor, the density of CD8+ cells in the center of the tumor, the density of CD3+ cells in the invasive margin, and the density of CD8+ cells in the invasive margin.

The density may be measured in the "cold spot", i.e., in the regions of the tumor sample where the density is the lowest, or in the 2, 3, 4, 5, 6, 7, 8, 9, 10 "cold spots", corresponding to the 2 to 10 area with the lowest densities.

The density may also be measured in the "hot spot", i.e., in the regions where the density is the highest, or in the 2, 3, 4, 5, 6, 7, 8, 9, 10 "hot spots", corresponding to the 2 to 10 area with the highest densities.

One can also determine the mean density on the whole tumor sample.

Typically, the method disclosed in WO2013186374 may be used for quantifying the immune cells in the tumor sample.

As used herein, the term "marker" consists of any detectable, measurable or quantifiable parameter that is indicative of the status of the adaptive immune response of the subject. A marker becomes a "biological marker" for the purpose of carrying the method of the present invention when a good statistical correlation is found between (i) an increase or a decrease of the quantification value for said marker and (ii) the survival time actually observed within patients. For calculating correlation values for each marker tested and thus determining the statistical relevance of said marker as a "biological marker" according to the invention, any one of the statistical method known by the one skilled in the art may be used. Illustratively, statistical methods using Kaplan-Meier curves and/or univariate analysis using the log-rank-test and/or a Cox proportional-hazards model may be used, as it is shown in the examples herein. Any marker for which a P value of less than 0.05, and even preferably less than $10^{-3}$, $10^{-4}$, $10^{-5}$, $10^{-6}$ or $10^{-7}$ (according to univariate and multivariate analysis (for example, log-rank test and Cox test, respectively) is determined consists of a "biological marker" useable in the cancer prognosis method of the invention. In some embodiments, the marker includes the presence of, or the number or density of, cells from the immune system. In some embodiments, the marker includes the presence of, or the amount of proteins specifically produced by cells from the immune system. In some embodiments, the marker includes the presence of, or the amount of, any biological material that is indicative of the level of genes related to the raising of a specific immune response of the host. Thus, in some embodiments, the marker includes the presence of, or the amount of, messenger RNA (mRNA) transcribed from genomic DNA encoding proteins which are specifically produced by cells from the immune system. In some embodiments, the marker includes surface antigens that are specifically expressed by cells from the immune system, including by B lymphocytes, T lymphocytes, monocytes/macrophages dendritic cells, NK cells, NKT cells, and NK-DC cells or alternatively mRNA encoding for said surface antigens. When performing method of the present invention with more than one biological marker, the number of distinct biological markers that are quantified at step a) are usually of less than 100 distinct markers, and in most embodiments of less than 50 distinct markers. The number of distinct biological markers that is necessary for obtaining an accurate and reliable prognosis, using the method of the present invention, may vary notably according to the type of technique for quantification. Illustratively, high statistical significance can be found with a combination of a small number of biological markers, when the method of the present invention is performed by in situ immunohistochemical detection of protein markers of interest, in particular when separate quantification of the said markers are performed both in the center of the tumor (CT) and in the invasive margin (IM). Illustratively, high statistical significance was obtained with only one marker or a combination of two markers, as disclosed in the EXAMPLE. Further illustratively, high statistical significance was also found with a small number of biological markers, when the method of the present invention is performed by gene expression analysis of gene markers of interest. Without wishing to be bound by any particular theory, the inventors believe that highly statistical relevance (P value lower than $10^{-3}$) is reached when method of the present invention is performed by using a gene expression analysis for biological marker quantification, and by using a combination of ten distinct biological markers, and more preferably a combination of fifteen distinct biological markers, most preferably twenty distinct biological markers, or more. In some embodiments, the level of 1; 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; or 50 markers is (are) determined. In the present specification, the name of each of the various biological markers of interest refers to the internationally recognised name of the corresponding gene, as found in internationally recognised gene sequences and protein sequences databases, including in the database from the HUGO Gene Nomenclature Committee. In the present specification, the name of each of the various biological markers of interest may also refer to the internationally recognised name of the corresponding gene, as found in the internationally recognised gene sequences and protein sequences database Genbank. Through these internationally recognised sequence databases, the nucleic acid and the amino acid sequences corresponding to each of the biological marker of interest described herein may be retrieved by the one skilled in the art.

The biological markers indicative of the status of the immune response may comprise the expression level of one or more genes or corresponding proteins listed in Table 9 of WO2007045996 which are: 18s, ACE, ACTB, AGTR1, AGTR2, APC, APOA1, ARF1, AXIN1, BAX, BCL2, BCL2L1, CXCR5, BMP2, BRCA1, BTLA, C3, CASP3, CASP9, CCL1, CCL11, CCL13, CCL16, CCL17, CCL18, CCL19, CCL2, CCL20, CCL21, CCL22, CCL23, CCL24, CCL25, CCL26, CCL27, CCL28, CCL3, CCL5, CCL7, CCL8, CCNB1, CCND1, CCNE1, CCR1, CCR10, CCR2, CCR3, CCR4, CCR5, CCR6, CCR7, CCR8, CCR9, CCRL2, CD154, CD19, CD1a, CD2, CD226, CD244, PDCD1LG1, CD28, CD34, CD36, CD38, CD3E, CD3G, CD3Z, CD4, CD40LG, CD5, CD54, CD6, CD68, CD69, CLIP, CD80, CD83, SLAMF5, CD86, CD8A, CDH1, CDH7, CDK2, CDK4, CDKN1A, CDKN1B, CDKN2A, CDKN2B, CEACAM1, COL4A5, CREBBP, CRLF2, CSF1, CSF2, CSF3, CTLA4, CTNNB1, CTSC, CX3CL1, CX3CR1, CXCL1, CXCL10, CXCL11, CXCL12, CXCL13, CXCL14, CXCL16, CXCL2, CXCL3, CXCL5, CXCL6, CXCL9, CXCR3, CXCR4, CXCR6, CYP1A2, CYP7A1, DCC, DCN, DEFA6, DICER1, DKK1, Dok-1, Dok-2, DOK6, DVL1, E2F4, EBI3, ECE1, ECGF1, EDN1, EGF, EGFR, EIF4E, CD105, ENPEP, ERBB2, EREG, FCGR3A,, CGR3B, FN1, FOXP3, FYN, FZD1, GAPD, GLI2, GNLY, GOLPH4, GRB2, GSK3B, GSTP1, GUSB, GZMA, GZMB, GZMH, GZMK, HLA-B, HLA-C, HLA-, MA, HLA-DMB, HLA-DOA, HLA-DOB, HLA-DPA1, HLA-DQA2, HLA-DRA, HLX1, HMOX1, HRAS, HSPB3, HUWE1, ICAM1, ICAM-2, ICOS, ID1, ifna1, ifna17, ifna2, ifna5, ifna6, ifna8, IFNAR1, IFNAR2, IFNG, IFNGR1, IFNGR2, IGF1, IHH, IKBKB, IL10, IL12A, IL12B, IL12RB1, IL12RB2, IL13, IL13RA2, IL15, IL15RA, IL17, IL17R, IL17RB, IL18, ILIA, IL1B, IL1R1, IL2, IL21, IL21R, IL23A, IL23R, IL24, IL27, IL2RA, IL2RB, IL2RG, IL3, IL31RA, IL4, IL4RA, IL5, IL6, IL7, IL7RA, IL8, CXCR1, CXCR2, IL9, IL9R, IRF1, ISGF3G, ITGA4, ITGA7, integrin alpha E (antigen CD103, human mucosal lymphocyte, antigen 1; alpha polypeptide), Gene hCG33203, ITGB3, JAK2, JAK3, KLRB1, KLRC4, KLRF1, KLRG1, KRAS, LAG3, LAIR2, LEF1, LGALS9, LILRB3, LRP2, LTA, SLAMF3, MADCAM1, MADH3, MADH7,MAF, MAP2K1, MDM2, MICA, MICB, MKI67, MMP12, MMP9, MTA1, MTSS1, MYC, MYD88, MYH6, NCAM1, NFATC1, NKG7, NLK, NOS2A, P2X7, PDCD1, PECAM-,, CXCL4, PGK1, PIAS1, PIAS2, PIAS3, PIAS4, PLAT, PML, PP1A, CXCL7, PPP2CA, PRF1, PROM1, PSMB5, PTCH, PTGS2, PTP4A3, PTPN6, PTPRC, RAB23, RAC/RHO, RAC2, RAF, RB1, RBL1, REN, Drosha, SELE, SELL, SELP, SERPINE1, SFRP1, SIRP beta 1, SKI, SLAMF1, SLAMF6, SLAMF7, SLAMF8, SMAD2, SMAD4, SMO, SMOH, SMURF1, SOCS1, SOCS2, SOCS3, SOCS4, SOCS5, SOCS6, SOCS7, SOD1, SOD2, SOD3, SOS1, SOX17, CD43, ST14, STAM, STAT1, STAT2, STAT3, STAT4, STAT5A, STAT5B, STAT6, STK36, TAP1, TAP2, TBX21, TCF7, TERT, TFRC, TGFA, TGFB1, TGFBR1, TGFBR2, TIM-3, TLR1, TLR10, TLR2, TLR3, TLR4, TLR5, TLR6, TLR7, TLR8, TLR9, TNF, TNFRSF10A, TNFRSF11A, TNFRSF18, TNFRSF1A, TNFRSF1B, OX-40, TNFRSF5, TNFRSF6, TNFRSF7, TNFRSF8, TNFRSF9, TNFSF10, TNFSF6, TOB1, TP53, TSLP, VCAM1, VEGF, WIF1, WNT1, WNT4, XCL1, XCR1, ZAP70 and ZIC2.

In the present specification, the name of each of the genes of interest refers to the internationally recognised name of the corresponding gene, as found in internationally recognised gene sequences and protein sequences databases, including the database from the HUGO Gene Nomenclature Committee. In the present specification, the name of each of the genes of interest may also refer to the internationally recognised name of the corresponding gene, as found in the internationally recognised gene sequences database Genbank. Through these internationally recognised sequence databases, the nucleic acid to each of the gene of interest described herein may be retrieved by one skilled in the art.

In a preferred embodiment, the biological markers indicative of the status of the immune response are those described in WO2014023706:

Under this embodiment, an expression level $EL_1$ of a single gene representative of human adaptive immune response and of a single gene representative of human immunosuppressive response (a pair of genes) is assessed in the method of the invention. Preferably one to three genes of each and more preferably one or two genes of each are used. A limited number of genes of each kind provides good and reliable results and is easy to implement. Particularly a single reference value is sufficient for each of both genes. The higher the number of genes, the more sophisticated is the reference value. Examples of determination of reference values are given thereafter.

The use of more genes than one or two pairs of genes is more difficult to implement and more expensive and time consuming but however provides other advantages. For example if the assessment of the expression level of one gene is erroneous, the overall result is compensated by the reserved of the other genes of the same kind (human adaptive immune response or immunosuppressive response).

As used herein the expression "gene representative of the adaptive immune response" refers to any gene that is expressed by a cell that is an actor of the adaptive immune response in the tumor or that contributes to the settlement of the adaptive immune response in the tumor. The adaptive immune response, also called "acquired immune response", comprises antigen-dependent stimulation of T cell subtypes, B cell activation and antibody production,. For example cells of the adaptive immune response include but are not limited to cytotoxic T cells, T memory T cells, Th1 and Th2 cells, activated macrophages and activated dendritic cells, NK cells and NKT cells. Accordingly, a gene representative of the adaptive immune response may be typically selected from the cluster of the co-modulated genes for the Th1 adaptive immunity, for the cytotoxic response, or for the memory response, and may encode for a Th1 cell surface marker, an interleukin (or an interleukin receptor), or a chemokine or (a chemokine receptor).

In a particular embodiment, the gene representative of the adaptive immune response is selected from the group consisting of

- the family of chemokines and chemokine receptors consisting of: CXCL13, CXCL9, CCL5, CCR2, CXCL10, CXCL11, CXCR3, CCL2 and CX3CL1,
- the family of cytokines consisting of: IL15,
- the TH1 family consisting of: IFNG, IRF1, STAT1, STAT4 and TBX21
- the family of lymphocytes membrane receptors consisting of: ITGAE, CD3D, CD3E, CD3G, CD8A, CD247, CD69 and ICOS,
- the family of cytotoxic molecules consisting of: GNLY, GZMH, GZMA, GZMB, GZMK, GZMM and PRF1,

and the kinase LTK.

Preferred such genes or corresponding proteins, because they provide the best results for the response of a patient to the treatment as shown hereafter in table 5, are reported in Table 1:

Table 1

| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |

As used herein the expression "gene representative of the immunosuppressive response" refers to any gene that is expressed by a cell that is an actor of the immunosuppressive response in the tumor or that contributes to the settlement of the immunosuppressive response in the tumor. For example, the immunosuppressive response comprises

- co-inhibition of antigen-dependent stimulation of T cell subtypes: genes CD276, CTLA4, PDCD1, CD274, TIM-3 or VTCN1 (B7H4),
- inactivation of macrophages and dendritic cells and inactivation of NK cells: genes TSLP, CD1A, or VEGFA
- expression of cancer stem cell marker, differentiation and/or oncogenesis: PROM1, IHH.
- expression of immunosuppressive proteins produced in the tumour environment: genes PF4, REN, VEGFA.

For example cells of the immunosuppressive response include immature dendritic cells (CD1A), regulatory T cells (Treg cells) and Th17 cells expressing IL17A gene.

Accordingly, a gene representative of the adaptive immune response may be typically selected from the group of the co-modulated adaptive immune genes, whereas the immunosuppressive genes, may be representative of the inactivation of immune cells (e.g. dendritic cells) and may contribute to induction of an immunosuppressive response.

In a particular embodiment, the gene or corresponding proteins representative of the immunosuppressive response is selected from the group consisting of genes reported in Table 2 hereunder:

Table 2

| |
|---|
| CD274 |
| CTLA4 |
| IHH |
| IL17A |

(continued)

| |
|---|
| PDCD1 |
| PF4 |
| PROM1 |
| REN |
| TIM-3 |
| TSLP |
| VEGFA |

Said genes are preferred because they provide the best results for the response of a patient to the treatment.

Under preferred conditions for implementing the invention, a gene representative of the adaptive immune response is selected from the group consisting of GNLY, CXCL13, CX3CL1, CXCL9, ITGAE, CCL5, GZMH, IFNG, CCR2, CD3D, CD3E, CD3G, CD8A, CXCL10, CXCL11, GZMA, GZMB, GZMK, GZMM, IL15, IRF1, LTK, PRF1, STAT1, CD69, CD247, ICOS, CXCR3, STAT4, CCL2 and TBX21 and a gene representative of the immunosuppressive response is selected from the group consisting of PF4, REN, VEGFA, TSLP, IL17A, PROM1, IHH, CD1A, CTLA4, PDCD1, CD276, CD274, TIM-3 and VTCN1 (B7H4).

Because some genes are more frequently found significant when combining one adaptive gene and one immunosuppressive gene, the most preferred genes are:

- genes representative of the adaptive immune response: CD3G, CD8A, CCR2 and GZMA
- genes representative of the immunosuppressive response: REN, IL17A, CTLA4 and PDCD1.

Under further preferred conditions for implementing the invention, a gene representative of the adaptive immune response and a gene representative of the immunosuppressive response are selected respectively from the groups consisting of the genes of Tables 1 and 2 above. Preferred combinations of two pairs of genes (total of 4 genes) are

- CCR2, CD3G, IL17A and REN and
- CD8A, CCR2, REN and PDCD1.

The precise choice of the genes selected for use in the present process may depend on the type of treatment contemplated for the patient. For example, genes selected from the group consisting of CX3CL1 IL15, CD247, CD3G, CD8A, PRF1, CCL5 and TBX21 for the immunosuppressive response, preferably CX3CL1 and IL15 and gene CTLA4 for the adaptive immune response will be preferred when a treatment using a drug such as monoclonal antibody working by activating the immune system such as Ipilimumab, also known as MDX-010 or MDX-101, marketed as Yervoy®, is contemplated for a patient.

Genes selected from the group consisting of IL15 and GZMA for the adaptive immune response, and gene VEGFA for the immunosuppressive response will be preferred when a treatment such as an antibody that inhibits vascular endothelial growth factor A (VEGF-A) such as bevacizumab marketed as Avastin®, is contemplated for a patient.

Similar considerations apply for example for the pair of genes GZMA - PDCD1 (also designated as CD279), when a treatment such as an antibody that targets PD-1 such as BMS-936558, is contemplated for a patient.

In a preferred embodiment, the biological markers indicative of the status of the immune response are those described in WO2014009535:

The biological markers indicative of the status of the immune response may comprise the expression level of one or more genes from the group consisting of CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2, and TBX21.

In a preferred embodiment, the biological markers indicative of the status of the immune response are those described in WO2012095448:

The biological markers indicative of the status of the immune response may comprise the expression level of one or more genes from the group consisting of GZMH, IFNG, CXCL13, GNLY, LAG3, ITGAE, CCL5, CXCL9, PF4, IL17A, TSLP, REN, IHH, PROM1 and VEGFA.

In a preferred embodiment, the biological markers indicative of the status of the immune response are those described in WO2012072750:

The biological markers indicative of the status of the immune response may comprise the expression level of a miRNA cluster comprising: miR.609, miR.518c, miR.520f, miR.220a, miR.362, miR.29a, miR.660, miR.603, miR.558, miR519b, miR.494, miR.130a, or miR.639.

**General methods for quantifying biological markers**

Any one of the methods known by the one skilled in the art for quantifying cellular types, a protein-type or an nucleic acid-type biological marker encompassed herein may be used for performing the cancer prognosis method of the invention. Thus any one of the standard and non-standard (emerging) techniques well known in the art for detecting and quantifying a protein or a nucleic acid in a sample can readily be applied.

Expression of a biological marker used in the invention may be assessed by any of a wide variety of well known methods for detecting expression of a transcribed nucleic acid or protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods.

In one preferred embodiment, expression of a marker is assessed using an antibody (e.g. a radio-labeled, chromophore-labeled, fluorophore-labeled, polymer-backbone-antibody, or enzyme-labeled antibody), an antibody derivative (e.g. an antibody conjugated with a substrate or with the protein or ligand of a protein-ligand pair {e.g. biotin-streptavidin}), or an antibody fragment (e.g. a single-chain antibody, an isolated antibody hypervariable domain, etc.) which binds specifically with a marker protein or fragment thereof, including a marker protein which has undergone all or a portion of its normal post-translational modification.

In certain embodiments, a biological marker, or a set of biological markers, may be quantified with any one of the immunohistochemistry methods known in the art.

Typically, for further analysis, one thin section of the tumor, is firstly incubated with labeled antibodies directed against one biological marker of interest. After washing, the labeled antibodies that are bound to said biological marker of interest are revealed by the appropriate technique, depending of the kind of label is borne by the labeled antibody, e.g. radioactive, fluorescent or enzyme label. Multiple labelling can be performed simultaneously.

Immunohistochemistry typically includes the following steps i) fixing the tumor tissue sample with formalin, ii) embedding said tumor tissue sample in paraffin, iii) cutting said tumor tissue sample into sections for staining, iv) incubating said sections with the binding partner specific for the immune checkpoint protein of interest, v) rinsing said sections, vi) incubating said section with a secondary antibody typically biotinylated and vii) revealing the antigen-antibody complex typically with avidin-biotin-peroxidase complex. Accordingly, the tumor tissue sample is firstly incubated with the binding partners having for the immune checkpoint protein of interest. After washing, the labeled antibodies that are bound to the immune checkpoint protein of interest are revealed by the appropriate technique, depending of the kind of label is borne by the labeled antibody, e.g. radioactive, fluorescent or enzyme label. Multiple labelling can be performed simultaneously. Alternatively, the method of the present invention may use a secondary antibody coupled to an amplification system (to intensify staining signal) and enzymatic molecules. Such coupled secondary antibodies are commercially available, e.g. from Dako, EnVision system. Counterstaining may be used, e.g. Hematoxylin & Eosin, DAPI, Hoechst. Other staining methods may be accomplished using any suitable method or system as would be apparent to one of skill in the art, including automated, semi-automated or manual systems.

For example, one or more labels can be attached to the antibody, thereby permitting detection of the target protein (i.e. the biological markers). Exemplary labels include radioactive isotopes, fluorophores, ligands, chemiluminescent agents, enzymes, and combinations thereof. Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke J R (1972) Annu. Rev. Biochem. 41:843-868. Affinity ligands can also be labeled with enzymes (e.g. horseradish peroxidase, alkaline phosphatase, beta-lactamase), radioisotopes (e.g. $^3$H, $^{14}$C, $^{32}$P, $^{35}$S or $^{125}$I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine. Various enzymatic staining methods are known in the art for detecting a protein of interest. For example, enzymatic interactions can be visualized using different enzymes such as peroxidase, alkaline phosphatase, or different chromogens such as DAB, AEC or Fast Red. In some embodiments, the label is a quantum dot. For example, Quantum dots (Qdots) are becoming increasingly useful in a growing list of applications including immunohistochemistry, flow cytometry, and plate-based assays, and may therefore be used in conjunction with this invention. Qdot nanocrystals have unique optical properties including an extremely bright signal for sensitivity and quantitation; high photostability for imaging and analysis. A single excitation source is needed, and a growing range of conjugates makes them useful in a wide range of cell-based applications. Qdot Bioconjugates are characterized by quantum yields comparable to the brightest traditional dyes available. Additionally, these quantum dot-based fluorophores absorb 10-1000 times more light than traditional dyes. The emission from the underlying Qdot quantum dots is narrow and symmetric which means overlap with other colors is minimized, resulting in minimal bleed through into adjacent detection channels and attenuated crosstalk, in spite of the fact that many more colors can be used simultaneously. In other examples, the antibody can be conjugated to peptides or proteins that can be detected via a labeled binding partner or antibody. In an indirect IHC assay, a secondary antibody or second binding partner is necessary to detect the binding of the first binding partner, as it is not labeled.

In some embodiments, the resulting stained specimens are each imaged using a system for viewing the detectable signal and acquiring an image, such as a digital image of the staining. Methods for image acquisition are well known to one of skill in the art. For example, once the sample has been stained, any optical or non-optical imaging device can be used to detect the stain or biomarker label, such as, for example, upright or inverted optical microscopes, scanning confocal microscopes, cameras, scanning or tunneling electron microscopes, canning probe microscopes and imaging infrared detectors. In some examples, the image can be captured digitally. The obtained images can then be used for quantitatively or semi-quantitatively determining the amount of the immune checkpoint protein in the sample, or the absolute number of cells positive for the maker of interest, or the surface of cells positive for the maker of interest. Various automated sample processing, scanning and analysis systems suitable for use with IHC are available in the art. Such systems can include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.). In particular, detection can be made manually or by image processing techniques involving computer processors and software. Using such software, for example, the images can be configured, calibrated, standardized and/or validated based on factors including, for example, stain quality or stain intensity, using procedures known to one of skill in the art (see e.g., published U.S. Patent Publication No. US20100136549). The image can be quantitatively or semi-quantitatively analyzed and scored based on staining intensity of the sample. Quantitative or semi-quantitative histochemistry refers to method of scanning and scoring samples that have undergone histochemistry, to identify and quantify the presence of the specified biomarker (i.e. immune checkpoint protein). Quantitative or semi-quantitative methods can employ imaging software to detect staining densities or amount of staining or methods of detecting staining by the human eye, where a trained operator ranks results numerically. For example, images can be quantitatively analyzed using a pixel count algorithms and tissue recognition pattern (e.g. Aperio Spectrum Software, Automated QUantitatative Analysis platform (AQUA® platform), or Tribvn with Ilastic and Calopix software), and other standard methods that measure or quantitate or semi-quantitate the degree of staining; see e.g., U.S. Pat. No. 8,023,714; U.S. Pat. No. 7,257,268; U.S. Pat. No. 7,219,016; U.S. Pat. No. 7,646,905; published U.S. Patent Publication No. US20100136549 and 20110111435; Camp et al. (2002) Nature Medicine, 8:1323-1327; Bacus et al. (1997) Analyt Quant Cytol Histol, 19:316-328). A ratio of strong positive stain (such as brown stain) to the sum of total stained area can be calculated and scored. The amount of the detected biomarker (i.e. the immune checkpoint protein) is quantified and given as a percentage of positive pixels and/or a score. For example, the amount can be quantified as a percentage of positive pixels. In some examples, the amount is quantified as the percentage of area stained, e.g., the percentage of positive pixels. For example, a sample can have at least or about at least or about 0, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 40%, 45%, 50%, 55%, 60%, 65%, 70%, 75%, 80%, 85%, 90%, 95% or more positive pixels as compared to the total staining area. For example, the amount can be quantified as an absolute number of cells positive for the maker of interest. In some embodiments, a score is given to the sample that is a numerical representation of the intensity or amount of the histochemical staining of the sample, and represents the amount of target biomarker (e.g., the immune checkpoint protein) present in the sample. Optical density or percentage area values can be given a scaled score, for example on an integer scale.

Thus, in some embodiments, the method of the present invention comprises the steps consisting in i) providing one or more immunostained slices of tissue section obtained by an automated slide-staining system by using a binding partner capable of selectively interacting with the biological marker, ii) proceeding to digitalisation of the slides of step i).by high resolution scan capture, iii) detecting the slice of tissue section on the digital picture iv) providing a size reference grid with uniformly distributed units having a same surface, said grid being adapted to the size of the tissue section to be analysed, and v) detecting, quantifying and measuring intensity or the absolute number of stained cells in each unit.

Multiplex tissue analysis techniques are particularly useful for quantifying several immune checkpoint proteins in the tumor tissue sample. Such techniques should permit at least five, or at least ten or more biomarkers to be measured from a single tumor tissue sample. Furthermore, it is advantageous for the technique to preserve the localization of the biomarker and be capable of distinguishing the presence of biomarkers in cancerous and non-cancerous cells. Such methods include layered immunohistochemistry (L-IHC), layered expression scanning (LES) or multiplex tissue immuno-noblotting (MTI) taught, for example, in U.S. Pat. Nos. 6,602,661, 6,969,615, 7,214,477 and 7,838,222; U.S. Publ. No. 2011/0306514; and in Chung & Hewitt, Meth Mol Biol, Prot Blotting Detect, Kurlen & Scofield, eds. 536: 139-148, 2009, each reference teaches making up to 8, up to 9, up to 10, up to 11 or more images of a tissue section on layered and blotted membranes, papers, filters and the like, can be used. Coated membranes useful for conducting the L-IHC/MTI process are available from 20/20 GeneSystems, Inc. (Rockville, MD).

In some embodiments, the L-IHC method can be performed on any of a variety of tissue samples, whether fresh or preserved. The samples included core needle biopsies that were routinely fixed in 10% normal buffered formalin and processed in the pathology department. Standard five μm thick tissue sections were cut from the tissue blocks onto charged slides that were used for L-IHC. Thus, L-IHC enables testing of multiple markers in a tissue section by obtaining copies of molecules transferred from the tissue section to plural bioaffinity- coated membranes to essentially produce copies of tissue "images." In the case of a paraffin section, the tissue section is deparaffinized as known in the art, for example, exposing the section to xylene or a xylene substitute such as NEO-CLEAR®, and graded ethanol solutions. The section can be treated with a proteinase, such as, papain, trypsin, proteinase K and the like. Then, a stack of a membrane substrate comprising, for example, plural sheets of a 10 μm thick coated polymer backbone with 0.4 μm diameter pores to channel tissue molecules, such as, proteins, through the stack, then is placed on the tissue section. The movement of fluid and tissue molecules is configured to be essentially perpendicular to the membrane surface. The sandwich of the section, membranes, spacer papers, absorbent papers, weight and so on can be exposed to heat to facilitate movement of molecules from the tissue into the membrane stack. A portion of the proteins of the tissue are captured on each of the bioaffinity-coated membranes of the stack (available from 20/20 GeneSystems, Inc., Rockville, MD). Thus, each membrane comprises a copy of the tissue and can be probed for a different biomarker using standard immunoblotting techniques, which enables open-ended expansion of a marker profile as performed on a single tissue section. As the amount of protein can be lower on membranes more distal in the stack from the tissue, which can arise, for example, on different amounts of molecules in the tissue sample, different mobility of molecules released from the tissue sample, different binding affinity of the molecules to the membranes, length of transfer and so on, normalization of values, running controls, assessing transferred levels of tissue molecules and the like can be included in the procedure to correct for changes that occur within, between and among membranes and to enable a direct comparison of information within, between and among membranes. Hence, total protein can be determined per membrane using, for example, any means for quantifying protein, such as, biotinylating available molecules, such as, proteins, using a standard reagent and method, and then revealing the bound biotin by exposing the membrane to a labeled avidin or streptavidin; a protein stain, such as, Blot fastStain, Ponceau Red, brilliant blue stains and so on, as known in the art.

In some embodiments, the present methods utilize Multiplex Tissue Imprinting (MTI) technology for measuring biomarkers, wherein the method conserves precious biopsy tissue by allowing multiple biomarkers, in some cases at least six biomarkers.

In some embodiments, alternative multiplex tissue analysis systems exist that may also be employed as part of the present invention. One such technique is the mass spectrometry-based Selected Reaction Monitoring (SRM) assay system ("Liquid Tissue" available from OncoPlexDx (Rockville, MD)). That technique is described in U.S. Pat. No. 7,473,532.

In some embodiments, the method of the present invention utilized the multiplex IHC technique developed by GE Global Research (Niskayuna, NY). That technique is described in U.S. Pub. Nos. 2008/0118916 and 2008/0118934. There, sequential analysis is performed on biological samples containing multiple targets including the steps of binding a fluorescent probe to the sample followed by signal detection, then inactivation of the probe followed by binding probe to another target, detection and inactivation, and continuing this process until all targets have been detected.

In some embodiments, multiplex tissue imaging can be performed when using fluorescence (e.g. fluorophore or Quantum dots) where the signal can be measured with a multispectral imagine system. Multispectral imaging is a technique in which spectroscopic information at each pixel of an image is gathered and the resulting data analysed with spectral image -processing software. For example, the system can take a series of images at different wavelengths that are electronically and continuously selectable and then utilized with an analysis program designed for handling such data. The system can thus be able to obtain quantitative information from multiple dyes simultaneously, even when the spectra of the dyes are highly overlapping or when they are co-localized, or occurring at the same point in the sample, provided that the spectral curves are different. Many biological materials auto fluoresce, or emit lower- energy light when excited by higher-energy light. This signal can result in lower contrast images and data. High-sensitivity cameras without multispectral imaging capability only increase the autofluorescence signal along with the fluorescence signal. Multispectral imaging can unmix, or separate out, autofluorescence from tissue and, thereby, increase the achievable signal-to-noise ratio. Briefly the quantification can be performed by following steps: i) providing a tumor tissue microarray (TMA) obtained from the patient, ii) TMA samples are then stained with anti-antibodies having specificity of the immune checkpoint protein(s) of interest, iii) the TMA slide is further stained with an epithelial cell marker to assist in automated segmentation of tumour and stroma, iv) the TMA slide is then scanned using a multispectral imaging system, v) the scanned images are processed using an automated image analysis software (e.g.Perkin Elmer Technology) which allows the detection, quantification and segmentation of specific tissues through powerful pattern recognition algorithms. The machine-learning algorithm was typically previously trained to segment tumor from stroma and identify cells labelled.

Determining an expression level of a gene in a tumor sample obtained from a patient can be implemented by a panel of techniques well known in the art.

Typically, an expression level of a gene is assessed by determining the quantity of mRNA produced by this gene.

Methods for determining a quantity of mRNA are well known in the art. For example nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The thus extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA), quantitative new generation sequencing of RNA (NGS).

Nucleic acids (s) comprising at least 10 nucleotides and exhibiting sequence complementarity or homology to the mRNA of interest herein find utility as hybridization probes or amplification primers. It is understood that such nucleic acids need not be completely identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500 nucleotides. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Nucleic acids which may be used as primers or probes in the above amplification and detection method may be assembled as a kit. Such a kit includes consensus primers and molecular probes. A preferred kit also includes the components necessary to determine if amplification has occurred. A kit may also include, for example, PCR buffers and enzymes; positive control sequences, reaction control primers; and instructions for amplifying and detecting the specific sequences.

In a particular embodiment, the expression of a biological marker used in the invention may be assessed by tagging the biomarker (in its DNA, RNA or protein for) with a digital oligonucleotide barcode, and to measure or count the number of barcodes.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

Probes made using the disclosed methods can be used for nucleic acid detection, such as in situ hybridization (ISH) procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pinkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am. J. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS®) can be directly optically detected when performing FISH. Alternatively, the probe can be labeled with a nonfluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT®) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publications Nos. 2006/0246524; 2006/0246523, and 2007/0117153.

It will be appreciated by those of skill in the art that by appropriately selecting labelled probe-specific binding agent pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can be labelled with a first hapten, such as biotin, while a second probe that corresponds to a second target sequence can be labelled with a second hapten, such as DNP. Following exposure of the sample to the probes, the bound probes can be detected by contacting the sample with a first specific binding agent (in this case avidin labelled with a first fluorophore, for example, a first spectrally distinct QUANTUM DOT®, e.g., that emits at 585 mn) and a second specific binding agent (in this case an anti-DNP antibody, or antibody fragment, labelled with a second fluorophore (for example, a second spectrally distinct QUANTUM DOT®, e.g., that emits at 705 mn). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

The expression level of a gene may be expressed as absolute expression level or normalized expression level. Both types of values may be used in the present method. The expression level of a gene is preferably expressed as normalized expression level when quantitative PCR is used as method of assessment of the expression level because small differences at the beginning of an experiment could provide huge differences after a number of cycles.

Typically, expression levels are normalized by correcting the absolute expression level of a gene by comparing its expression to the expression of a gene that is not relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows comparing the expression level of one sample, e.g., a patient sample, with the expression level of another sample, or comparing samples from different sources.

In the present specification, the name of each of the genes of interest refers to the internationally recognised name of the corresponding gene, as found in internationally recognised gene sequences and protein sequences databases, including the database from the HUGO Gene Nomenclature Committee. In the present specification, the name of each of the genes of interest may also refer to the internationally recognised name of the corresponding gene, as found in the internationally recognised gene sequences database Genbank. Through these internationally recognised sequence databases, the nucleic acid to each of the gene of interest described herein may be retrieved by one skilled in the art.

The cancer prognosis method of the invention may be performed with a combination of genes provided that the combination comprises at least one one gene representative of the adaptive immune response and at least one gene representative of the immunosuppressive response. The number of genes that may be used in the present method is only limited by the number of distinct biological genes of interest that are practically available at the time of carrying out the method. Accordingly, in one embodiment, a combination of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50 distinct genes are quantified, preferably a combination of 2, 3, 4, 5, 6, 7, 8, 9, or 10 genes and more preferably a combination of 2, 3, 4, 5, or 6, genes. However, the number of combined genes that are required for reaching a high statistical relevance (e.g. P value lower than $10^{-3}$), will be depending on the technique used for quantifying the combination of genes. The number of genes used as genes representative of the adaptive immune response and the number of genes used as genes representative of the immunosuppressive response may be the same or different.

Under preferred conditions of implementation of the invention, an about balanced number of genes of each kind (adaptive immune response and immunosuppressive response) is used, for example 2 of each, or three of each, or 5 of one kind and 6 of the other kind.

Determining an expression level of a gene in a tumor sample obtained from a patient can be implemented by a panel of techniques well known in the art.

Typically, an expression level of a gene is assessed by determining the quantity of mRNA produced by this gene. A subject of the present application is therefore a a method for screening patients with a cancer defined above comprising determining an expression level ELA of one or several genes representative of human adaptive immune response or the expression level ELI of one or several genes representative of human immunosuppressive response by determining the quantity of mRNA corresponding to said genes.

Methods for determining a quantity of mRNA are well known in the art. For example nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The thus extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA), quantitative new generation sequencing of RNA (NGS).

Nucleic acids (s) comprising at least 10 nucleotides and exhibiting sequence complementarity or homology to the mRNA of interest herein find utility as hybridization probes or amplification primers. It is understood that such nucleic acids need not be completely identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500 nucleotides. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Nucleic acids which may be used as primers or probes in the above amplification and detection method may be assembled as a kit. Such a kit includes consensus primers and molecular probes. A preferred kit also includes the components necessary to determine if amplification has occurred. A kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

Probes made using the disclosed methods can be used for nucleic acid detection, such as in situ hybridization (ISH) procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pinkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am. J. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS®) can be directly optically detected when performing FISH. Alternatively, the probe can be labeled with a nonfluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT®) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publications Nos. 2006/0246524; 2006/0246523, and 2007/0117153.

It will be appreciated by those of skill in the art that by appropriately selecting labelled probe-specific binding agent pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can be labelled with a first hapten, such as biotin, while a second probe that corresponds to a second target sequence can be labelled with a second hapten, such as DNP. Following exposure of the sample to the probes, the bound probes can be detected by contacting the sample with a first specific binding agent (in this case avidin labelled with a first fluorophore, for example, a first spectrally distinct QUANTUM DOT®, e.g., that emits at 585 mn) and a second specific binding agent (in this case an anti-DNP antibody, or antibody fragment, labelled with a second fluorophore (for example, a second spectrally distinct QUANTUM DOT®, e.g., that emits at 705 mn). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

The expression level of a gene may be expressed as absolute expression level or normalized expression level. Both types of values may be used in the present method. The expression level of a gene is preferably expressed as normalized expression level when quantitative PCR is used as method of assessment of the expression level because small differences at the beginning of an experiment could provide huge differences after a number of cycles.

Typically, expression levels are normalized by correcting the absolute expression level of a gene by comparing its expression to the expression of a gene that is not relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows comparing the expression level of one sample, e.g., a patient sample, with the expression level of another sample, or comparing samples from different sources.

**Anti tumoral treatments**

The present method therefore allows defining inter alia a new group of patients which had never been identified until now, i.e. patients whose cancer will be successfully treated by an anti-cancer treatment.

An anti-cancer treatment may consist of radiotherapy, chemotherapy or immunotherapy. The treatment may consist of an adjuvant therapy (i.e. treatment after chirurgical resection of the primary tumor) of a neoadjuvant therapy (i.e. treatment before chirurgical resection of the primary tumor).

The present invention therefore relates to a chemotherapeutic agent, a radiotherapeutic agent, or an immunotherapeutic agent, preferably the latter, for use in the treatment of a stage I-III cancer patient who has been considered as a good responder to antitumoral treatment according to the above method of the invention.

The term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin;

duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chrommomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK®; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.].) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and phannaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and phannaceutically acceptable salts, acids or derivatives of any of the above.

The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively the immunotherapeutic treatment may consist of administering the patient with an amount of immune cells (T cells, NK, cells, dendritic cells, B cells...).

Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents.

Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors.

Interferons (IFNs) contemplated by the present invention include the common types of IFNs, IFN-alpha (IFN-a), IFN-beta (IFN-beta) and IFN-gamma (IFN-y). IFNs can act directly on cancer cells, for example, by slowing their growth, promoting their development into cells with more normal behaviour and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). The use of IFN-alpha, alone or in combination with other immunotherapeutics or with chemotherapeutics, has shown efficacy in the treatment of various cancers including melanoma (including metastatic melanoma), renal cancer (including metastatic renal cancer), breast cancer, prostate cancer, and cervical cancer (including metastatic cervical cancer).

Interleukins contemplated by the present invention include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present invention. Interleukins, alone or in combination with other immunotherapeutics or with chemotherapeutics, have shown efficacy in the treatment of various cancers including renal cancer (including metastatic renal cancer), melanoma (including metastatic melanoma), ovarian cancer (including recurrent ovarian cancer), cervical cancer (including metastatic cervical cancer), breast cancer, colorectal cancer, lung cancer, brain cancer, and prostate cancer.

Interleukins have also shown good activity in combination with IFN-alpha in the treatment of various cancers (Negrier et al., Ann Oncol. 2002 13(9):1460-8 ; Touranietal, J. Clin. Oncol. 2003 21(21):398794).

Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in patients undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Colony stimulating factors have shown efficacy in the treatment of cancer, including melanoma, colorectal cancer (including metastatic colorectal cancer), and lung cancer.

Non-cytokine adjuvants suitable for use in the combinations of the present invention include, but are not limited to, Levamisole, alum hydroxide (alum), Calmette-Guerin bacillus (ACG), incomplete Freund's Adjuvant (IFA), QS-21, DETOX, Keyhole limpet hemocyanin (KLH) and dinitrophenyl (DNP). Non-cytokine adjuvants in combination with other immuno- and/or chemotherapeutics have demonstrated efficacy against various cancers including, for example, colon cancer and colorectal cancer (Levimasole); melanoma (BCG and QS-21); renal cancer and bladder cancer (BCG).

In addition to having specific or non-specific targets, immunotherapeutic agents can be active, i.e. stimulate the body's own immune response, or they can be passive, i.e. comprise immune system components that were generated external to the body.

Passive specific immunotherapy typically involves the use of one or more monoclonal antibodies that are specific for a particular antigen found on the surface of a cancer cell or that are specific for a particular cell growth factor. Monoclonal antibodies may be used in the treatment of cancer in a number of ways, for example, to enhance a subject's immune response to a specific type of cancer, to interfere with the growth of cancer cells by targeting specific cell growth factors, such as those involved in angiogenesis, or by enhancing the delivery of other anticancer agents to cancer cells when linked or conjugated to agents such as chemotherapeutic agents, radioactive particles or toxins.

Monoclonal antibodies currently used as cancer immunotherapeutic agents that are suitable for inclusion in the combinations of the present invention include, but are not limited to, rituximab (Rituxan®), trastuzumab (Herceptin®), ibritumomab tiuxetan (Zevalin®), tositumomab (Bexxar®), cetuximab (C-225, Erbitux®), bevacizumab (Avastin®), gemtuzumab ozogamicin (Mylotarg®), alemtuzumab (Campath®), and BL22. Monoclonal antibodies are used in the treatment of a wide range of cancers including breast cancer (including advanced metastatic breast cancer), colorectal cancer (including advanced and/or metastatic colorectal cancer), ovarian cancer, lung cancer, prostate cancer, cervical cancer, melanoma and brain tumours. Other examples include anti-CTLA4 antibodies (e.g. Ipilimumab), anti-PDI antibodies, anti-PDLI antibodies, anti-TIM3 antibodies, anti-LAG3 antibodies, anti-B7H3 antibodies, anti-B7H4 antibodies or anti-B7H6 antibodies.

Monoclonal antibodies can be used alone or in combination with other immunotherapeutic agents or chemotherapeutic agents.

Active specific immunotherapy typically involves the use of cancer vaccines. Cancer vaccines have been developed that comprise whole cancer cells, parts of cancer cells or one or more antigens derived from cancer cells. Cancer vaccines, alone or in combination with one or more immuno- or chemotherapeutic agents are being investigated in the treatment of several types of cancer including melanoma, renal cancer, ovarian cancer, breast cancer, colorectal cancer, and lung cancer. Non-specific immunotherapeutics are useful in combination with cancer vaccines in order to enhance the body's immune response.

The immunotherapeutic treatment may consist of an adoptive immunotherapy as described by Nicholas P. Restifo, Mark E. Dudley and Steven A. Rosenberg "Adoptive immunotherapy for cancer: harnessing the T cell response, Nature Reviews Immunology, Volume 12, April 2012). In adoptive immunotherapy, the patient's circulating lymphocytes, or tumor infiltrated lymphocytes, are isolated in vitro, activated by lymphokines such as IL-2 or transuded with genes for tumor necrosis, and readministered (Rosenberg et al., 1988; 1989). The activated lymphocytes are most preferably be the patient's own cells that were earlier isolated from a blood or tumor sample and activated (or "expanded") in vitro. This form of immunotherapy has produced several cases of regression of melanoma and renal carcinoma.

The term "radiotherapeutic agent" as used herein, is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

In a preferred embodiment, the antitumoral treatment is a treatment with a checkpoint blockade cancer immunotherapy agent.

As used herein, the expression " checkpoint blockade cancer immunotherapy agent" or "immune checkpoint inhibitor" (both expressions will be used interchangeably) has its general meaning in the art and refers to any compound inhibiting the function of an immune inhibitory checkpoint protein. Inhibition includes reduction of function and full blockade.

Preferred immune checkpoint inhibitors are antibodies that specifically recognize immune checkpoint proteins. A number of immune checkpoint inhibitors are known and in analogy of these known immune checkpoint protein inhibitors, alternative immune checkpoint inhibitors may be developed in the (near) future. The immune checkpoint inhibitors include peptides, antibodies, nucleic acid molecules and small molecules. In particular, the immune checkpoint inhibitor used in the present invention is administered for enhancing the proliferation, migration, persistence and/or cytoxic activity of CD8+ T cells in the subject and in particular the tumor-infiltrating of CD8+ T cells of the subject. As used herein "CD8+ T cells" has its general meaning in the art and refers to a subset of T cells which express CD8 on their surface. They are MHC class I-restricted, and function as cytotoxic T cells. "CD8+ T cells" are also called CD8+ T cells are called cytotoxic T lymphocytes (CTL), T-killer cell, cytolytic T cells, CD8+ T cells or killer T cells. CD8 antigens are members of the immunoglobulin supergene family and are associative recognition elements in major histocompatibility complex class I-restricted interactions. The ability of the immune checkpoint inhibitor to enhance T CD8 cell killing activity may be determined by any assay well known in the art. Typically said assay is an in vitro assay wherein CD8+ T cells are brought into contact with target cells (e.g. target cells that are recognized and/or lysed by CD8+ T cells). For example, the immune checkpoint inhibitor used in the present invention can be selected for the ability to increase specific lysis by CD8+ T cells by more than about 20%, preferably with at least about 30%, at least about 40%, at least about 50%, or more of the specific lysis obtained at the same effector: target cell ratio with CD8+ T cells or CD8 T cell lines that are contacted by the immune checkpoint inhibitor used in the present invention, Examples of protocols for classical cytotoxicity assays are conventional.

Typically, the checkpoint blockade cancer immunotherapy agent is an agent which blocks an immunosuppressive receptor expressed by activated T lymphocytes, such as cytotoxic T lymphocyte-associated protein 4 (CTLA4) and programmed cell death 1 (PDCD1, best known as PD-1), or by NK cells, like various members of the killer cell immunoglobulin-like receptor (KIR) family, or an agent which blocks the principal ligands of these receptors, such as PD-1 ligand CD274 (best known as PD-L1 or B7-H1).

Typically, the checkpoint blockade cancer immunotherapy agent is an antibody.

In some embodiments, the checkpoint blockade cancer immunotherapy agent is an antibody selected from the group consisting of anti-CTLA4 antibodies, anti-PDI antibodies, anti-PDLI antibodies, anti-PDL2 antibodies, anti-TIM-3 antibodies, anti-LAG3 antibodies, anti-IDOI antibodies, anti-TIGIT antibodies, anti-B7H3 antibodies, anti-B7H4 antibodies, anti-BTLA antibodies, and anti-B7H6 antibodies.

Examples of anti-CTLA-4 antibodies are described in US Patent Nos: 5,811,097; 5,811,097; 5,855,887; 6,051,227; 6,207,157; 6,682,736; 6,984,720; and 7,605,238. One anti-CDLA-4 antibody is tremelimumab, (ticilimumab, CP-675,206). In some embodiments, the anti-CTLA-4 antibody is ipilimumab (also known as 10D1, MDX-D010) a fully human monoclonal IgG antibody that binds to CTLA-4.

Examples of PD-1 and PD-L1 antibodies are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699. In some embodiments, the PD-1 blockers include anti-PD-LI antibodies. In certain other embodiments the PD-1 blockers include anti-PD-1 antibodies and similar binding proteins such as nivolumab (MDX 1106, BMS 936558, ONO 4538), a fully human IgG4 antibody that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ;

AMP-224 is a fusion protein of B7-DC; an antibody Fc portion; BMS-936559 (MDX- 1105-01) for PD-L1 (B7-H1) blockade.

Other immune-checkpoint inhibitors include lymphocyte activation gene-3 (LAG-3) inhibitors, such as IMP321, a soluble Ig fusion protein (Brignone et al., 2007, J. Immunol. 179:4202-4211).

Other immune-checkpoint inhibitors include B7 inhibitors, such as B7-H3 and B7-H4 inhibitors. In particular, the anti-B7-H3 antibody MGA271 (Loo et al., 2012, Clin. Cancer Res. July 15 (18) 3834).

Also included are TIM3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. The natural ligand of TIM-3 is galectin 9 (Gal9). Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signaling pathway and/or block the binding of TIM-3 to galectin-9. Antibodies having specificity for TIM-3 are well known in the art and typically those described in WO2011155607, WO2013006490 and WO2010117057.In some embodiments, the immune checkpoint inhibitor is an Indoleamine 2,3-dioxygenase (IDO) inhibitor, preferably an IDOI inhibitor. Examples of IDO inhibitors are described in WO 2014150677. Examples of IDO inhibitors include without limitation 1-methyl-tryptophan (IMT), $\beta$- (3-benzofuranyl)-alanine, $\beta$ -(3-benzo(b)thienyl)-alanine), 6-nitro-tryptophan, 6- fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a $\beta$-carboline derivative or a brassilexin derivative. Preferably the IDO inhibitor is selected from 1-methyl-tryptophan, $\beta$-(3-benzofuranyl)-alanine, 6-nitro-L-tryptophan, 3-Amino-naphtoic acid and $\beta$-[3-benzo(b)thienyl] -alanine or a derivative or prodrug thereof.

In some embodiments, the immune checkpoint inhibitor is an anti-TIGIT (T cell immunoglobin and ITIM domain) antibody.

In a preferred embodiment, the checkpoint blockade cancer immunotherapy agent is a CTLA4 blocking antibody, such as Ipilimumab, or a PD-1 blocking antibody, such as Nivolumab or Pembrolizumab, or a combination thereof.

The invention will be further illustrated by the following figures and example. These example and figures should not be interpreted in any way as limiting the scope of the present invention.

## FIGURES

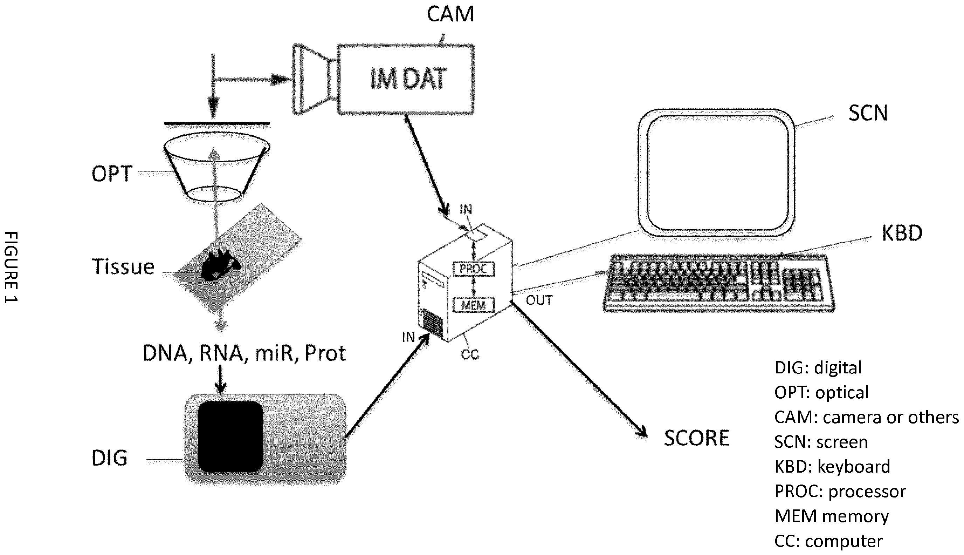

Figure 1 is a schematic illustration of a system for carrying out the invention.

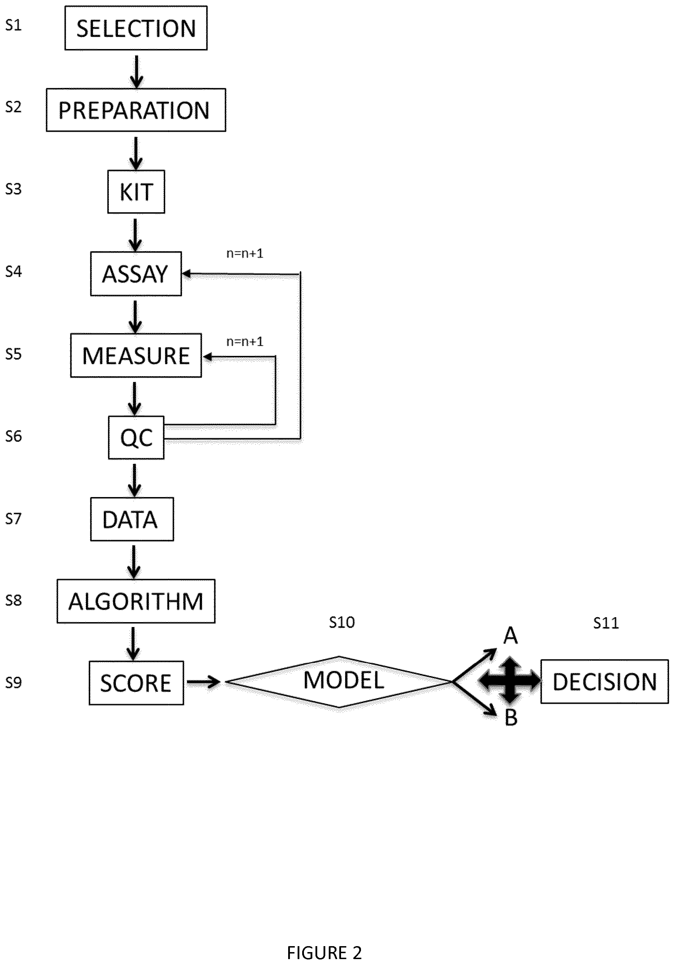

Figure 2 is a flow diagram illustrating one example of a procedure for implementing the invention;
Figure 3 represents a Kaplan Meir curve for 5 predetermined reference arithmetic mean values of percentile.

## EXAMPLE

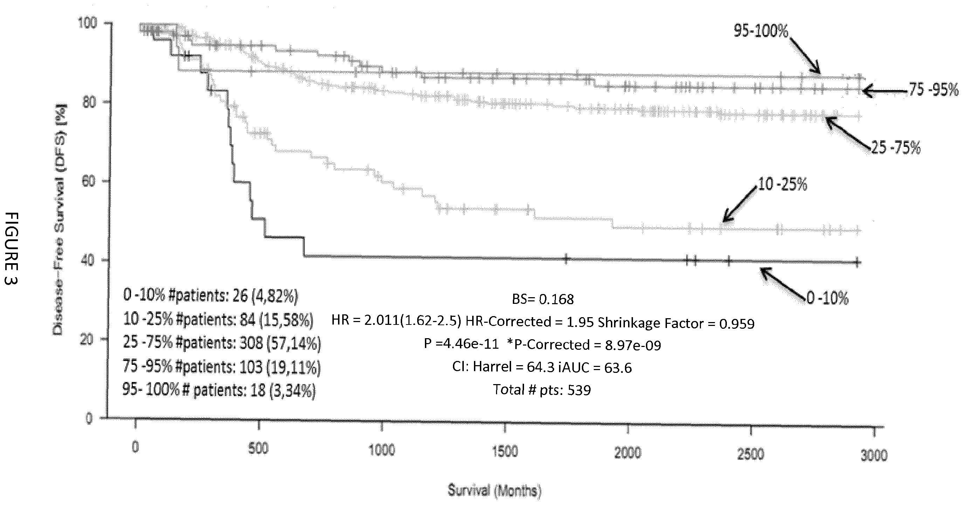

A reference group of patients with Stage I/II/III colorectal cancer (n=539) were analyzed accordingly to the algorithmic score method. Whole slide tumors were stained with CD3 and CD8 antibodies. Using digital pathology and image analysis software, the invasive margin (IM) of the tumor and the core (center) (CT) the tumor were determined. The densities of CD3 and CD8 in CT and IM regions were quantified (cells/mm$^2$). The distribution of all the densities or all the markers is plotted and reported into a table. The corresponding percentiles (i.e. with 5% steps interval) were derived. The mean over the 4 density biomarker values was calculated with a weight of 1 for each biomarker, in the example illustrated in the table below. The Kaplan Meier curves for such cohort is illustrated on Figure 3, with 5 groups categories (I0, I1, I2, I3, I4), corresponding to the mean percentile values of 0-10%, 10-25%, 25-75%, 75-95%, 95-100%, respectively.

In the following tables is an illustration of an example of calculation of the arithmetic mean value of percentiles for a hypothetical patient:

| hypothetical patient | CD3CT-mean | CD3IM-mean | CD8CT-mean | CD8IM-mean |
|---|---|---|---|---|
| | 390 | 900 | 50 | 340 |

| percentile | CD3CT-mean | CD3IM-mean | CD8CT-mean | CD8IM-mean |
|---|---|---|---|---|
| 5% | 112 | 230 | 24 | 46 |
| 10% | 165 | 302 | 31 | 65 |
| 15% | 205 | 385 | 40 | 95 |
| 20% | 244 | 435 | 48 | 113 |
| 25% | 287 | 488 | 60 | 130 |
| 30% | 324 | 556 | 69 | 152 |
| 35% | 364 | 620 | 77 | 175 |
| 40% | 397 | 702 | 89 | 196 |
| 45% | 416 | 757 | 103 | 222 |
| 50% | 448 | 795 | 117 | 252 |
| 55% | 497 | 877 | 135 | 282 |
| 60% | 541 | 1000 | 151 | 317 |
| 65% | 611 | 1074 | 176 | 357 |
| 70% | 686 | 1193 | 206 | 415 |
| 75% | 746 | 1340 | 234 | 456 |
| 80% | 818 | 1449 | 263 | 528 |
| 85% | 934 | 1615 | 306 | 621 |
| 90% | 1078 | 1807 | 405 | 749 |
| 95% | 1343 | 2163 | 604 | 1058 |
| 100% | >1343 | >2163 | >604 | >1058 |

The arithmetic mean value of percentile for the hypothetical patient would be about 47.5. The hypothetical patient would be classified as belonging to the group 12.

The same type of experiments have been performed with the 21 biological markers which are the expression levels of the 21 genes from the group consisting of CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2, and TBX21 and with the 15 biological markers which are the expression levels of the 15 genes from the group consisting of GZMH, IFNG, CXCL13, GNLY, LAG3, ITGAE, CCL5, CXCL9, PF4, IL17A, TSLP, REN, IHH, PROM1 and VEGFA.

Very accurate results have been obtained by calculating the median value of percentile.

**REFERENCES:**

Throughout this application, various references describe the state of the art to which this invention pertains.

**Claims**

1. An *in vitro* method for the prognosis of the survival time of a patient suffering from a solid cancer, which method comprises the following steps:

22

a) detecting and quantifying two or more biological markers indicative of the status of the immune response of said patient against said cancer, wherein each biological marker indicative of the status of the immune response is detected *in vitro* and quantified in a tumor sample obtained from said patient;

b) comparing each values obtained at step a) for said two or more biological markers with a distribution of values obtained for each of said two or more biological markers from a reference group of patients suffering from said cancer;

c) determining for each values obtained at step a) for said two or more biological markers the percentile of the distribution to which the values obtained at step a) correspond;

d) calculating the arithmetic mean value or the median value of percentiles of the biological markers determined in step c); and

e) comparing the arithmetic mean value or the median value of percentiles obtained at step d) with a predetermined reference arithmetic mean value or a predetermined median value of percentiles, which predetermined reference value is correlated with survival time.

2. An *in vitro* method for assessing the responsiveness of a patient suffering from a solid cancer to an antitumoral treatment, which method comprises the following steps:

a) detecting and quantifying two or more biological markers indicative of the status of the immune response of said patient against said cancer, wherein each biological marker indicative of the status of the immune response is detected *in vitro* and quantified in a tumor sample obtained from said patient;

b) comparing each values obtained at step a) for said two or more biological markers with a distribution of values obtained for each of said two or more biological markers from a reference group of patients suffering from said cancer;

c) determining for each values obtained at step a) for said two or more biological markers the percentile of the distribution to which the values obtained at step a) correspond;

d) calculating the arithmetic mean value or the median value of percentiles of the biological markers determined in step c); and

e) comparing the arithmetic mean value or the median value of percentiles obtained at step d) with a predetermined reference arithmetic mean value or a predetermined median value of percentiles, which predetermined reference value is correlated with response to said antitumoral treatment.

3. The method according to claim 1 or 2, wherein the solid cancer is a colorectal cancer, a breast cancer, a lung cancer, a head and neck cancer, bladder cancer, a ovary cancer, or a prostate cancer.

4. The method according to claim 3, wherein the solid cancer is a colorectal cancer.

5. The method according to any of claims 1 to 4, wherein the two or more biological markers comprise the cell density of cells from the immune system.

6. The method according to claim 5, wherein the two or more biological markers comprise the density of CD3+ cells, the density of CD8+ cells, the density of CD45RO+ cells, the density of GZM-B+ cells, the density of B cells, and/or the density of DC cells.

7. The method according to claim 6, wherein the two or more biological markers comprise the density of CD3+ cells and the density of CD8+ cells, the density of CD3+ cells and the density of CD45RO+ cells, the density of CD3+ cells the density of GZM-B+ cells, the density of CD8+ cells and the density of CD45RO+ cells, the density of CD8+ cells and the density of GZM-B+ cells; or the density of CD45RO+ cells and the density of GZM-B+ cells.

8. The method according to any of claims 5 to 7, wherein the density of cells from the immune system are quantified in the center of the tumor and/or in the invasive margin of the tumor.

9. The method according to claim 8, wherein the two or more biological markers comprise the density of CD3+ cells in center of the tumor, the density of CD8+ cells in the center of the tumor, the density of CD3+ cells in the invasive margin, and the density of CD8+ cells in the invasive margin.

10. The method according to any of claims 5 to 9, wherein the density is measured in a region of the tumor sample where the density is the lowest.

**11.** The method according to any of claims 5 to 9, wherein the density is measured in a region of the tumor sample where the density is the highest.

**12.** The method according to any of claims 1 to 11, wherein the two or more biological markers comprise the expression level of one or more genes from the group consisting of CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2, and TBX21.

**13.** The method according to any of claims 1 to 12, wherein the two or more biological markers comprise the expression level of one or more genes from the group consisting of GZMH, IFNG, CXCL13, GNLY, LAG3, ITGAE, CCL5, CXCL9, PF4, IL17A, TSLP, REN, IHH, PROM1 and VEGFA.

**14.** The method according to any of claims 1 to 12, wherein the two or more biological markers comprise an expression level of at least one gene representative of human adaptive immune response and an expression level of at least one gene representative of human immunosuppressive response.

**15.** The method according to claim 14, wherein the at least one gene representative of human adaptive immune response is selected from the group consisting of

| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |

and said at least one gene representative of human immunosuppressive response is selected from the group consisting of:

| |
|---|
| CD274 |
| CTLA4 |
| IHH |
| IL17A |
| PDCD1 |

(continued)

| |
|---|
| PF4 |
| PROM1 |
| REN |
| TIM-3 |
| TSLP |
| VEGF |

16. The method according to any one of the preceding claims, wherein each biological marker is a nucleic acid.

17. The method according to any one of the preceding claims, wherein each biological marker is detected by mRNA hybridization and/or amplification.

18. The method according to any one of claims 1 to 15, wherein each biological marker is a protein.

19. The method according to any one of claims 1 to 15 and 18, wherein each biological marker is detected by immunohistochemistry.

**Patentansprüche**

1. Ein *in vitro* Verfahren zur Prognose der Überlebenszeit eines Patienten mit einem soliden Krebs, wobei das Verfahren die folgenden Schritte umfasst:

   a) Detektieren und Quantifizieren von zwei oder mehr biologischen Markern, die den Status der Immunantwort von besagtem Patienten gegen besagten Krebs anzeigen, wobei jeder biologische Marker, der den Status der Immunantwort anzeigt, in einer von besagtem Patienten erhaltenen Tumorprobe *in vitro* detektiert und quantifiziert ist;
   b) Vergleichen der einzelnen Werte, die in Schritt a) für besagte zwei oder mehr biologische Marker erhalten sind, mit einer Verteilung von Werten, die für jeden der besagten zwei oder mehr biologischen Marker von einer Patientenreferenzgruppe mit besagtem Krebs erhalten sind;
   c) Bestimmen für die einzelnen Werte, die in Schritt a) für besagte zwei oder mehr biologische Marker erhalten sind, des Perzentils der Verteilung, dem die in Schritt a) erhaltenen Werten entsprechen;
   d) Berechnen des arithmetischen Mittelwerts oder des Medianwerts von in Schritt c) bestimmten Perzentilen der biologischen Marker; und
   e) Vergleichen des in Schritt d) erhaltenen arithmetischen Mittelwerts oder Medianwerts von Perzentilen mit einem vorbestimmten arithmetischen Referenzmittelwert oder einem vorbestimmten Medianwert von Perzentilen, wobei der vorbestimmte Referenzwert mit der Überlebenszeit korreliert ist.

2. Ein *in vitro* Verfahren zur Beurteilung des Ansprechens eines Patienten mit einem soliden Krebs auf eine Antitumorbehandlung, wobei das Verfahren die folgenden Schritte umfasst:

   a) Detektieren und Quantifizieren von zwei oder mehr biologischen Markern, die den Status der Immunantwort von besagtem Patienten gegen besagten Krebs anzeigen, wobei jeder biologische Marker, der den Status der Immunantwort anzeigt, in einer von besagtem Patienten erhaltenen Tumorprobe *in vitro* detektiert und quantifiziert ist;
   b) Vergleichen der einzelnen Werte, die in Schritt a) für besagte zwei oder mehr biologische Marker erhalten sind, mit einer Verteilung von Werten, die für jeden der besagten zwei oder mehr biologischen Marker von einer Patientenreferenzgruppe mit besagtem Krebs erhalten sind;
   c) Bestimmen für die einzelnen Werte, die in Schritt a) für besagte zwei oder mehr biologische Marker erhalten sind, des Perzentils der Verteilung, dem die in Schritt a) erhaltenen Werten entsprechen;
   d) Berechnen des arithmetischen Mittelwerts oder des Medianwerts von in Schritt c) bestimmten Perzentilen der biologischen Marker; und
   e) Vergleichen des in Schritt d) erhaltenen arithmetischen Mittelwerts oder Medianwerts von Perzentilen mit einem vorbestimmten arithmetischen Referenzmittelwert oder einem vorbestimmten Medianwert von Perzentilen, wobei der vorbestimmte Referenzwert mit der Antwort auf besagte Antitumorbehandlung korreliert ist.

3. Das Verfahren gemäß Anspruch 1 oder 2, wobei der solide Krebs ein Kolorektalkrebs, ein Brustkrebs, ein Lungenkrebs, ein Kopf- und Halskrebs, Blasenkrebs, ein Eierstockkrebs oder ein Prostatakrebs ist.

4. Das Verfahren gemäß Anspruch 3, wobei der solide Krebs ein Kolorektalkrebs ist.

5. Das Verfahren gemäß einem der Ansprüche 1 bis 4, wobei die zwei oder mehr biologischen Marker die Zelldichte von Zellen des Immunsystems umfassen.

6. Das Verfahren gemäß Anspruch 5, wobei die zwei oder mehr biologischen Marker die Dichte von CD3+-Zellen, die Dichte von CD8+-Zellen, die Dichte von CD45RO+-Zellen, die Dichte von GZM-B+-Zellen, die Dichte von B-Zellen und/oder die Dichte von DC-Zellen umfassen.

7. Das Verfahren gemäß Anspruch 6, wobei die zwei oder mehr biologischen Marker die Dichte von CD3+-Zellen und die Dichte von CD8+-Zellen, die Dichte von CD3+-Zellen und die Dichte von CD45RO+-Zellen, die Dichte von CD3+-Zellen und die Dichte von GZM-B+-Zellen, die Dichte von CD8+-Zellen und die Dichte von CD45RO+-Zellen, die Dichte von CD8+-Zellen und die Dichte von GZM-B+-Zellen; oder die Dichte von CD45RO+-Zellen und die Dichte von GZM-B+-Zellen umfassen.

8. Das Verfahren gemäß einem der Ansprüche 5 bis 7, wobei die Dichte von Zellen des Immunsystems im Zentrum des Tumors und/oder im invasiven Rand des Tumors quantifiziert ist.

9. Das Verfahren gemäß Anspruch 8, wobei die zwei oder mehr biologischen Marker die Dichte von CD3+-Zellen im Zentrum des Tumors, die Dichte von CD8+-Zellen im Zentrum des Tumors, die Dichte von CD3+-Zellen im invasiven Rand und die Dichte von CD8+-Zellen im invasiven Rand umfassen.

10. Das Verfahren gemäß einem der Ansprüche 5 bis 9, wobei die Dichte in einem Bereich der Tumorprobe gemessen ist, wo die Dichte am geringsten ist.

11. Das Verfahren gemäß einem der Ansprüche 5 bis 9, wobei die Dichte in einem Bereich der Tumorprobe gemessen ist, wo die Dichte am höchsten ist.

12. Das Verfahren gemäß einem der Ansprüche 1 bis 11, wobei die zwei oder mehr biologischen Marker das Expressionslevel von zwei oder mehr Genen aus der Gruppe umfassen, bestehend aus CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2 und TBX21.

13. Das Verfahren gemäß einem der Ansprüche 1 bis 12, wobei die zwei oder mehr biologischen Marker das Expressionslevel von zwei oder mehr Genen aus der Gruppe umfassen, bestehend aus GZMH, IFNG, CXCL13, GNLY, LAG3, ITGAE, CCL5, CXCL9, PF4, IL17A, TSLP, REN, IHH, PROM1 und VEGFA.

14. Das Verfahren gemäß einem der Ansprüche 1 bis 12, wobei die zwei oder mehr biologischen Marker ein Expressionslevel wenigstens eines Gens, das für eine adaptive humane Immunantwort repräsentativ ist, und ein Expressionslevel wenigstens eines Gens, das für eine immunsuppressive humane Immunantwort repräsentativ ist, umfassen.

15. Das Verfahren nach Anspruch 14, wobei das wenigstens eine Gen, das für eine adaptive humane Immunantwort repräsentativ ist, aus der Gruppe ausgewählt ist, bestehend aus:

| CCL5 |
|------|
| CCR2 |
| CD247 |
| CD3E |

(fortgesetzt)

| |
|---|
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |

und besagtes wenigstens eine Gen, das für eine immunsuppressive humane Immunantwort repräsentativ ist, aus der Gruppe ausgewählt ist, bestehend aus:

| |
|---|
| CD274 |
| CTLA4 |
| IHH |
| IL17A |
| PDCD1 |
| PF4 |
| PROM1 |
| REN |
| TIM-3 |
| TSLP |
| VEGF |

16. Das Verfahren gemäß einem der vorhergehenden Ansprüche, wobei jeder biologische Marker eine Nukleinsäure ist.

17. Das Verfahren gemäß einem der vorhergehenden Ansprüche, wobei jeder biologische Marker mittels mRNA-Hybridisierung und/oder -Amplifizierung detektiert ist.

18. Das Verfahren gemäß einem der Ansprüche 1 bis 15, wobei jeder biologische Marker ein Protein ist.

19. Das Verfahren gemäß einem der Ansprüche 1 bis 15 und 18, wobei jeder biologische Marker immunhistochemisch detektiert ist.

**Revendications**

1. Méthode *in vitro* pour pronostiquer le temps de survie d'un patient souffrant d'un cancer solide, laquelle méthode comprend les étapes suivantes :

   a) détection et quantification de deux, ou plus, marqueurs biologiques indicatifs du statut de la réponse immunitaire dudit patient contre ledit cancer, dans lesquelles chaque marqueur biologique du statut de la réponse immunitaire est détecté *in vitro* et quantifié dans un échantillon de tumeur obtenu dudit patient ;
   b) comparaison de chacune des valeurs obtenues à l'étape a) pour lesdits deux, ou plus, marqueurs biologiques avec une distribution de valeurs obtenues pour chacun desdits deux, ou plus, marqueurs biologiques provenant d'un groupe de référence de patients souffrant dudit cancer ;
   c) détermination, pour chacune des valeurs obtenues à l'étape a) pour lesdits deux, ou plus, marqueurs biologiques, du percentile de la distribution auquel correspondent les valeurs obtenues à l'étape a) ;
   d) calcul de la valeur arithmétique moyenne ou de la valeur médiane des percentiles des marqueurs biologiques déterminés à l'étape c) ; et
   e) comparaison de la valeur arithmétique moyenne ou de la valeur médiane des percentiles obtenue à l'étape d) avec une valeur arithmétique moyenne de référence prédéterminée ou une valeur médiane prédéterminée de percentiles, laquelle valeur de référence prédéterminée est corrélée au temps de survie.

2. Méthode *in vitro* pour déterminer la sensibilité d'un patient souffrant d'un cancer solide à un traitement antitumoral, laquelle méthode comprend les étapes suivantes :

   a) détection et quantification de deux, ou plus, marqueurs biologiques indicatifs du statut de la réponse immunitaire dudit patient contre ledit cancer, dans lesquelles chaque marqueur biologique du statut de la réponse immunitaire est détecté *in vitro* et quantifié dans un échantillon de tumeur obtenu dudit patient ;
   b) comparaison de chacune des valeurs obtenues à l'étape a) pour lesdits deux, ou plus, marqueurs biologiques avec une distribution de valeurs obtenues pour chacun desdits deux, ou plus, marqueurs biologiques provenant d'un groupe de référence de patients souffrant dudit cancer ;
   c) détermination, pour chacune des valeurs obtenues à l'étape a) pour lesdits deux, ou plus, marqueurs biologiques, du percentile de la distribution auquel correspondent les valeurs obtenues à l'étape a) ;
   d) calcul de la valeur arithmétique moyenne ou de la valeur médiane des percentiles des marqueurs biologiques, déterminés à l'étape c) ; et
   e) comparaison de la valeur arithmétique moyenne ou de la valeur médiane des percentiles obtenue à l'étape d) avec une valeur arithmétique moyenne de référence prédéterminée ou une valeur médiane prédéterminée de percentiles, laquelle valeur de référence prédéterminée est corrélée à la réponse audit traitement antitumoral.

3. Méthode selon la revendication 1 ou 2, dans laquelle le cancer solide est un cancer colorectal, un cancer du sein, un cancer du poumon, un cancer de la tête et du cou, un cancer de la vessie, un cancer ovarien, ou un cancer de la prostate.

4. Méthode selon la revendication 3, dans laquelle le cancer solide est un cancer colorectal.

5. Méthode selon l'une quelconque des revendications 1 à 4, dans laquelle les deux, ou plus, marqueurs biologiques comprennent la densité cellulaire de cellules du système immunitaire.

6. Méthode selon la revendication 5, dans laquelle les deux, ou plus, marqueurs biologiques comprennent la densité de cellules CD3+, la densité des cellule CD8+, la densité de cellules CD45RO+, la densité de cellules GZM-B+, la densité de cellules B, et/ou la densité de cellules DC.

7. Méthode selon la revendication 6, dans laquelle les deux, ou plus, marqueurs biologiques comprennent la densité de cellules CD3+ et la densité de cellules CD8+, la densité de cellules CD3+ et la densité de cellules CD45RO+, la densité de cellules CD3+ et la densité de cellules GZM-B+, la densité de cellules CD8+ et la densité de cellules CD45RO+, la densité de cellules CD8+ et la densité de cellules GZM-B+ ; ou la densité de cellules CD45RO+ et la densité de cellules GZM-B+.

8. Méthode selon l'une quelconque des revendications 5 à 7, dans laquelle la densité des cellules du système immunitaire est quantifiée dans le centre de la tumeur et/ou dans la marge invasive de la tumeur.

9. Méthode selon la revendication 8, dans laquelle les deux, ou plus, marqueurs biologiques comprennent la densité de cellules CD3+ au centre de la tumeur, la densité de cellules CD8+ au centre de la tumeur, la densité de cellules CD3+ dans le contour invasif, et la densité de cellules CD8+ dans le contour invasif.

10. Méthode selon l'une quelconque des revendications 5 à 9, dans laquelle la densité est mesurée dans une région de l'échantillon de tumeur où la densité est la plus faible.

11. Méthode selon l'une quelconque des revendications 5 à 9, dans laquelle la densité est mesurée dans une région de l'échantillon de tumeur où la densité est la plus forte.

12. Méthode selon l'une quelconque des revendications 1 à 11, dans laquelle les deux, ou plus, marqueurs biologiques comprennent le niveau d'expression d'un ou plusieurs gènes du groupe constitué par CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2, et TBX21.

13. Méthode selon l'une quelconque des revendications 1 à 12, dans laquelle les deux, ou plus, marqueurs biologiques comprennent le niveau d'expression d'un ou plusieurs gènes du groupe constitué par GZMH, IFNG, CXCL13, GNLY, LAG3, ITGAE, CCL5, CXCL9, PF4, IL17A, TSLP, REN, IHH, PROM1 et VEGFA.

14. Méthode selon l'une quelconque des revendications 1 à 12, dans laquelle les deux, ou plus, marqueurs biologiques comprennent le niveau d'expression d'au moins un gène représentatif de la réponse immunitaire adaptative humaine et le niveau d'expression d'au moins un gène représentatif de la réponse immunodépressive humaine.

15. Méthode selon la revendication 14, dans laquelle l'au moins un gène représentatif de la réponse immunitaire adaptative humaine est choisi dans le groupe constitué par

| CCL5 |
|---|
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |

et ledit au moins un gène représentatif d'une réponse immunodépressive est choisi dans le groupe constitué par :

| CD274 |
|---|
| CTLA4 |
| IHH |
| IL17A |
| PDCD1 |
| PF4 |
| PROM1 |
| REN |
| TIM-3 |
| TSLP |
| VEGF |

**16.** Méthode selon l'une quelconque des revendications précédentes, dans laquelle chaque marqueur biologique est un acide nucléique.

**17.** Méthode selon l'une quelconque des revendications précédentes, dans laquelle chaque marqueur biologique est détecté par hybridation et/ou amplification d'ARNm.

**18.** Méthode selon l'une quelconque des revendications 1 à 15, dans laquelle chaque marqueur biologique est une protéine.

**19.** Méthode selon l'une quelconque des revendications 1 à 15 et 18, dans laquelle chaque marqueur biologique est détecté par immunohistochimie.

FIGURE 1

DIG: digital
OPT: optical
CAM: camera or others
SCN: screen
KBD: keyboard
PROC: processor
MEM memory
CC: computer

SCN

KBD

CAM

IM DAT

OUT

PROC

MEM

IN

CC

IN

SCORE

OPT

Tissue

DNA, RNA, miR, Prot

DIG

FIGURE 2

FIGURE 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015007625 A
- WO 2014023706 A
- WO 2014009535 A
- WO 2013186374 A
- WO 2013107907 A
- WO 2013107900 A
- WO 2012095448 A
- WO 2012072750 A
- WO 2007045996 A
- WO 2010120814 A
- WO 2004000992 A
- US 8068988 B
- US 20100136549 A
- US 8023714 B
- US 7257268 B
- US 7219016 B
- US 7646905 B
- US 20110111435 A
- US 6602661 B
- US 6969615 B
- US 7214477 B
- US 7838222 B
- US 20110306514 A
- US 7473532 B
- US 20080118916 A
- US 20080118934 A
- US 4888278 A
- US 5447841 A
- US 5472842 A
- US 5427932 A
- US 6942970 B
- US 6280929 B
- US 20060246524
- US 20060246523 A
- US 20070117153 A
- US 5811097 A
- US 5855887 A
- US 6051227 A
- US 6207157 B
- US 6682736 B
- US 6984720 B
- US 7605238 B
- US 7488802 B
- US 7943743 B
- US 8008449 B
- US 8168757 B
- US 8217149 B
- WO 03042402 A
- WO 2008156712 A
- WO 2010089411 A
- WO 2010036959 A
- WO 2011066342 A
- WO 2011159877 A
- WO 2011082400 A
- WO 2011161699 A
- WO 2011155607 A
- WO 2013006490 A
- WO 2010117057 A
- WO 2014150677 A

**Non-patent literature cited in the description**

- **GALON et al.** Cancer classification using the Immunoscore: a worldwide task force. *J Transl Med.,* 2012, vol. 10, 205
- **GALON et al.** *Immunity,* 2013, vol. 39, 11-26
- **OLLI et al.** Human Molecular Genetics. Elsevier, 2001
- **KONONEN et al.** *Nature Medicine,* vol. 1198
- **STRYER L.** *Science,* 1968, vol. 162, 526-533
- **BRAND L ; GOHLKE J R.** *Annu. Rev. Biochem.,* 1972, vol. 41, 843-868
- **CAMP et al.** *Nature Medicine,* 2002, vol. 8, 1323-1327
- **BACUS et al.** *Analyt Quant Cytol Histol,* 1997, vol. 19, 316-328
- **CHUNG ; HEWITT.** Meth Mol Biol, Prot Blotting Detect. 2009, vol. 536, 139-148
- **PINKEL et al.** *Proc. Natl. Acad. Sci.,* 1986, vol. 83, 2934-2938
- **PINKEL et al.** *Proc. Natl. Acad. Sci.,* 1988, vol. 85, 9138-9142
- **LICHTER et al.** *Proc. Natl. Acad. Sci.,* 1988, vol. 85, 9664-9668
- **TANNER et al.** *Am. J. Pathol.,* 2000, vol. 157, 1467-1472
- **HOHEISEL.** *Nature Reviews, Genetics,* 2006, vol. 7, 200-210
- Agnew Chem Intl. Ed. Engl. 1994, vol. 33, 183-186

- **NEGRIER et al.** *Ann Oncol.,* 2002, vol. 13 (9), 1460-8
- **TOURANIETAL, J.** *Clin. Oncol.,* 2003, vol. 21 (21), 398794
- **NICHOLAS P. RESTIFO ; MARK E. DUDLEY ; STEVEN A. ROSENBERG.** Adoptive immunotherapy for cancer: harnessing the T cell response. *Nature Reviews Immunology,* April 2012, vol. 12
- **BRIGNONE et al.** *J. Immunol.,* 2007, vol. 179, 4202-4211
- **LOO et al.** *Clin. Cancer Res.,* 15 July 2012, vol. 18, 3834
- **FOURCADE et al.** *J. Exp. Med.,* 2010, vol. 207, 2175-86
- **SAKUISHI et al.** *J. Exp. Med.,* 2010, vol. 207, 2187-94